# EUROPEAN PATENT APPLICATION

(11) **EP 3 521 412 A1**
(43) Date of publication of application: **07.08.2019**
(21) Application number: 17856028.0
(22) Date of filing: 25.09.2017
(51) Int. Cl.: C12M 1/12, B01D 63/02, C12M 1/26, C12N 1/02, C12N 5/0783, C12N 5/0784, A61K 35/15, A61K 35/17, A61P 37/04

(54) **TOOL FOR ASEPTICALLY TREATING SUSPENSION**

(30) Priority: 27.09.2016 JP 2016188166
(71) Applicant: Yonemitsu, Yoshikazu, Fukuoka 8100023 (JP); GAIA BioMedicine Inc., Chuo-ku Fukuoka-City Fukuoka 810-0023 (JP)
(72) Inventor: YONEMITSU Yoshikazu, Fukuoka 8100023 (JP); HARADA Yui, Fukuoka 8190395 (JP)
(74) Representative: Vossius & Partner Patentanwälte Rechtsanwälte mbB
(86) International application number: PCT/JP2017/034463
(87) International publication number: WO 2018/062075

(57) **Abstract**

The object is to provide a means for aseptically separating a suspending medium from a suspension. There is provided a device comprising at least the followings: a hollow fiber type separator for separating a liquid A from a suspension comprising an inlet and outlet for passing the suspension, which communicate with the inside of hollow fibers, and at least one discharge port for discharging the separated liquid A, which communicates with the outside of the hollow fibers; an aseptic connection connector for aseptically connecting a bag containing the suspension in a non-aseptic environment, which is connected to the inlet of the hollow fiber type separator; a drainage bag for storing the separated liquid A, which is connected to the discharge port of the hollow fiber type separator; and a collection bag for storing a suspensoid from which the liquid A has been separated, which is connected to the inlet or outlet of the hollow fiber type separator.

## Description

### Technical Field

The present invention relates to an aseptic treatment of a suspension in which particles are dispersed in a liquid, for example, aseptic replacement of culture medium contained in a cell suspension with another liquid. The present invention is useful in the fields of life science, medical science, manufacture of cells for therapeutic use, and so forth.

### Background Art

For preparing cells for therapeutic use used for treatment of cancer, or the like by administering them to a patient, in many cases, it is first required to separate only necessary cells from a cell population collected from a living body. The obtained cells are usually subjected to culture steps in vitro for induction, proliferation, activation etc., but when they are administered to a patient, the components originating from the culture medium must be removed.

The invention described in Patent document 1 was accomplished for the purpose of providing an apparatus for easily concentrating leukocyte components including stem cells from blood in a short period of time by using a suction operation, not using gravity separation. It proposes, with paying attention to the deformability of erythrocytes, an apparatus for concentrating leukocyte components containing stem cells using a membrane filter having micropores which allows erythrocytes, plasma, etc. to pass through, and collects components including leukocytes as non-filterable components. It is described that, according to this apparatus, there can be constituted a leukocyte fraction collection system circuit for collecting leukocytes by back washing using plasma with a pathway of plasma bag-leukocyte capturing apparatus-collection bag, and a syringe provided in the circuit as a means for transporting blood can be used as a driving power source, so that the apparatus can be used in a standing state, and position change such as inversion for upside-down installation is not required.

As a method for producing a cell concentrate in a short period of time by a simple method without loss of cells and serious damages to the cells, Patent document 2 proposes a method for producing a cell concentrate using a cell suspension processing apparatus of internal pressure filtration type, which comprises a cell suspension inlet, a filtrate outlet, a cell suspension outlet, and a hollow fiber type separation membrane disposed between the cell suspension inlet and the cell suspension outlet, wherein average internal pore diameter of pores of the hollow fiber type separation membrane is 0.1 to 10 µm, and a value obtained by dividing initial filtration rate for the filtrate with linear velocity of the cell suspension flowing through the inside of the hollow fibers is 2.5 or smaller. It describes that, according to this method, the cells can be highly efficiently collected while removing contaminants other than the cells in the suspension such as proteins, and the cell concentrate can be produced with reducing clogging of the hollow fibers with the cells. It also describes that since it does not use a method for suppressing clogging based on reverse washing of the membrane, or the like, which imposes loads on the cells, damages on the cells are reduced to make the cell survival rate higher, and since the processing can be performed in an aseptic closed system, the concentrated cells can be provided as those for cell therapies.

### Prior Art References

### Patent documents

Patent document 1: Japanese Patent Unexamined Publication (KOKAI) No. 2012-139112 (Japanese Patent No. 5785713)
Patent document 2: International Patent Publication WO2013/061859

### Summary of the Invention]

### Object to be Achieved by the Invention

In order to prepare cells for therapeutic use, investigations must be done not only from the aspect of efficiency, but also from the aspect of ensuring quality and safety. Further, if such cells can be efficiently prepared by simple operations in general medical facilities without any requirements of special equipments, dramatic increase of the number of patients who can enjoy such therapies can be expected. The inventors of the present invention conducted many researches and developments concerning techniques of manufacturing of products for regeneration medicine etc. (cell therapy, gene therapy etc.), established practically usable NK cell GMP production techniques, and advanced final drug product designing. During this process, they came to pay attention to the importance of operations in a completely closed system.

Since highly active NK cells cannot be frozen and thawed in a state that they maintain the activities thereof, NK cells transported in a frozen state are required first to be subjected to a culture step for re-activation thereof before administration to a patient. This culture step must be aseptically performed. Then, culture medium components must be removed from the cultured cells, and the obtained cells must be re-suspended in lactated Ringer's solution or the like suitable for drip infusion administration at an appropriate cell density. Although the culture medium can be removed by precipitating the cells in a centrifugation tube by a centrifugation technique, and removing the culture medium as a supernatant, this operation must be aseptically performed, therefore it must be performed in a clean room, and thus there is a problem that such cells for administration to patients can be prepared only in facilities having special equipments and skillful technical experts.

If the operations of culturing once frozen cells, and at the time of use in therapy, removing culture medium from the cultured cells, as well as washing the cells and suspending the cells in drip, as required, can be performed in a completely closed circuit, the safety as cells for therapeutic use is enhanced, and in addition, they can be performed under a non-aseptic environment such as general medical facilities.

If there is a simple system that can aseptically treat not only cell culture liquid, but also a suspension, application thereof to various situations other than the preparation of cells for therapeutic use can also be expected.

### Means for Achieving the Object

The inventors of the present invention conducted various researches concerning a method for performing a treatment of aseptically removing a suspending medium from a suspension containing particles such as cells. As a result, they found that if a hollow fiber type filter is used, and a suspension is passed thorough the hollow fibers, the liquid part of the suspension as a suspending medium can be efficiently removed from the particles as a suspensoid, and a system using such hollow fibers is a completely closed system, and can be constituted as a circuit suitable for practical use not requiring electric pump for liquid feeding, and accomplished the present invention.

The present invention provides the followings.
[1] A device for aseptically separating a liquid A from a suspension containing the liquid A as a suspending medium and a suspensoid, the device comprising at least the followings:
   - a hollow fiber type separator for separating the liquid A from the suspension, the hollow fiber type separator comprising
      an inlet and outlet for passing the suspension, which communicate with the inside of hollow fibers, and
      at least one discharge port for discharging the separated liquid A, which communicates with the outside of the hollow fibers;
   - an aseptic connection connector for aseptically connecting a bag containing the suspension in a non-aseptic environment, which is connected to the inlet of the hollow fiber type separator;
   - a drainage bag for storing the separated liquid A, which is connected to the discharge port of the hollow fiber type separator; and
   - a collection bag for storing the suspensoid from which the liquid A has been separated, which is connected to the inlet or outlet of the hollow fiber type separator.
[2] The device according to 1, which further comprises the following:
   - at least one bag containing liquid B, which contains a liquid B for washing or collecting the suspensoid from which the suspending medium has been separated, and is connected to the inlet or outlet of the hollow fiber type separator.
[3] The device according to 2, which comprises two bags containing liquid B.
[4] The device according to 2 or 3, wherein a first bag containing liquid B is connected to the inlet of the hollow fiber type separator, and a second bag containing liquid B is connected to the outlet of the hollow fiber type separator.
[5] The device according to any one of 1 to 4, which further comprises:
   - a first clamp for closing the outlet of the hollow fiber type separator, and
   - a second clamp for closing the discharge port of the hollow fiber type separator.
[6] The device according to any one of 1 to 5, wherein:
   the device is for aseptically separating a culture medium from a cell suspension containing cells as the suspensoid and the culture medium as the liquid A, and
   the hollow fibers of the hollow fiber type separator have such an internal diameter that the cell suspension can pass through them, and such a pore diameter that the cells and the culture medium can be separated.
[7] The device according to 6, wherein:
   the cells are cells for therapeutic use to be intravenously administered, the device is for aseptically substituting a liquid suitable for intravenous administration for the culture medium of the cell suspension;
   the device comprises a first bag containing liquid B connected to the inlet of the hollow fiber type separator, and a second bag containing liquid B connected to the outlet of the hollow fiber type separator, and the liquids B is the liquid suitable for intravenous administration.
[8] A kit for preparing cells for therapeutic use, the kit comprising the followings:
   - the device according to 7; and
   - a bag containing stored cells, which contains useful cells together with a preservation liquid, and provided with at least one aseptic connection connector.
[9] The kit according to 8, which further comprises the following:
   - a bag containing culture medium, which contains a cell culture medium.
[10] The kit according to 8 or 9, wherein the cells are any cells selected from the group consisting of dendritic cells, NK cells, NKT cells, activated T cells, and CAR-T cells.
[11] A method for preparing useful cells aseptically separated from a liquid A from a suspension containing the liquid A as a suspending medium and the useful cells, the method comprising the following steps:
   (1) the step of aseptically connecting a bag containing suspension in which the suspension is retained to the aseptic connection connector of the device according to any one of 1 to 7;
   (2) the step of feeding the suspension into the hollow fiber type separator of which outlet is closed from the inlet of the hollow fiber type separator to separate the liquid A, so that the separated liquid A is stored in the drainage bag, and the useful cells from which the liquid A has been separated remain in the inside of the hollow fibers; and
   (3) the step of feeding a liquid B prepared beforehand into the hollow fiber type separator of which discharge port is closed from the outlet of the hollow fiber type separator to extrude the useful cells in the inside of the hollow fibers, and storing them in the collection bag.
[12] A method for preparing cells for therapeutic use from a cell suspension, the method comprising the following steps:
   (1) the step of aseptically connecting a bag containing cell suspension in which a cell suspension is retained to the aseptic connection connector of the device according to 7;
   (2) the step of feeding the cell suspension into the hollow fiber type separator of which outlet is closed from the inlet of the hollow fiber type separator to separate the culture medium, so that the separated culture medium is stored in the drainage bag, and the cells from which the culture medium has been separated remain in the inside of the hollow fibers;
   (3) the step of feeding a liquid suitable for intravenous administration from the first bag containing liquid B into the hollow fiber type separator of which outlet is closed to wash the cells remained in the inside of the hollow fibers, from which cells the culture medium has been separated, and
   (4) the step of feeding a liquid suitable for intravenous administration from the second bag containing liquid B to the hollow fiber type separator of which discharge port is closed to extrude the washed cells in the inside of the hollow fibers, and storing them in the collection bag to obtain the cells for therapeutic use.
[13] The method according to 12, which further comprises the following step as a step preceding the step (1):
   (0) the step of aseptically adding a culture medium to a cell storage bag containing frozen cells together with a preservation liquid to obtain a bag containing cell suspension, which retains the cell suspension.
[14] The method according to 13, wherein the cells are any cells selected from the group consisting of dendritic cells, NK cells, NKT cells, activated T cells, and CAR-T cells.

### Effect of the Invention

According to the present invention, such a treatment as removal of a suspending medium from a suspension, washing of suspensoid, and re-suspension of suspensoid in another suspending medium can be aseptically performed even in a non-aseptic environment. Further, according to the present invention, such a treatment as mentioned above can be performed without using electric machinery.

### Brief Description of the Drawings

- [Fig. 1]: Examples of the device and kit of the present invention.
- [Fig. 2]: An example of the device of the present invention during a treatment.
- [Fig. 3]: Drawings showing injection of cells and collection of cells.
- [Fig. 4]: A graph showing collection ratio.

### Modes for Carrying out the Invention

The ranges of numerical values indicated as "X to Y" include the numerical values of X and Y as the minimum and maximum values, unless especially indicated. The expression "A and/or B" means at least one of A and B, unless especially indicated. Preparation method is production method in other words.

### <Suspension, suspensoid and suspending medium (liquid A) as objects of treatment>

Various kinds of suspensions can be treated with the device of the present invention. Suspension refers to solid and liquid in a state that the solid is dispersed in the liquid, and contains a suspensoid (solid dispersoid) and a suspending medium (liquid dispersion medium). Dispersion may not be uniform.

The device of the present invention is used for aseptically separating a liquid A from a suspension containing the liquid A as a suspending medium and a suspensoid. The suspensoid may consist of particles of various kinds, shapes, and sizes, and are not particularly limited. However, the present invention can be preferably applied to particles acceptable as drugs, cosmetics, or foods, for example, nanoparticles consisting of polymer micelles used as DDS, fine particles used for cosmetics, cultured cells, cells for therapeutic use, and so forth. Particularly preferred examples include floating animal cells (henceforth animal cells may be simply referred to as "cells"), and further preferred examples include cells used for a treatment (prophylactic treatment, therapeutic treatment, etc.) of a disease or condition in a human or nonhuman animal (cells for therapeutic use). In the following descriptions, the present invention and embodiments thereof may be explained for a case where the device of the present invention is for treating cells for therapeutic use as an example, but those skilled in the art can appropriately apply the explanations to other cases, and understand such embodiments of the present invention.

The liquid A as a suspending medium may consist of any of various kinds of liquids suitable for the suspensoid. When the suspensoid is floating cells, the liquid A is, for example, a liquid for preserving cells, or a culture medium. Examples of the liquid for preserving cells include culture media containing an ingredient for protecting cells from freezing injury, for example, DMSO, glycerol, serum, etc., as required. The culture medium can be appropriately chosen depending on the type of the cells from, for example, DMEM, EMEM, RPMI-1640, α-MEM, F-12, F-10, M-199 and so forth, which are known as basal media for animal cell culture, and used. The culture medium may contain an ingredient for proliferation, induction of differentiation, and activation of cells. Examples of such an ingredient include serum, cytokines, growth factors, and hormones, and specific examples include insulin, transferrin, serum albumin, IL-2, and so forth.

The volumes of the suspension and liquid A can be appropriately determined depending on the purpose. When the device of the present invention is for treating cells for therapeutic use, the suspension may contain cells in a number of 10⁷ to 10⁹ order, and the volume thereof may be 10 mL to 1 L.

### <Device and kit>

The present invention provides an device for aseptically separating a liquid A from a suspension containing the liquid A as a suspending medium and a suspensoid, which comprises at least the followings:
- a hollow fiber type separator for separating the liquid A from the suspension comprising
   at least one discharge port for discharging the separated liquid A, which communicates with the outside of hollow fibers; and
   an inlet and outlet for passing the suspension, which communicate with the inside of the hollow fibers,
- an aseptic connection connector for aseptically connecting a bag containing the suspension in a non-aseptic environment, which is connected to the inlet of the hollow fiber type separator;
- a drainage bag for storing the separated liquid A, which is connected to the discharge port of the hollow fiber type separator; and
- a collection bag for storing the suspensoid from which the liquid A has been separated, which is connected to the inlet or outlet of the hollow fiber type separator.

The present invention also provides a kit for preparing cells for therapeutic use comprising the following part A and part B:
part A: a part consisting of the device of the present invention, and
part B: a part consisting of a bag containing cell suspension (7) provided with at least one aseptic connection connector (4a, 9a) and containing useful cells together with a preservation liquid or culture medium.

The kit may further contain the following part C:
part C: a part consisting of a bag containing culture medium (8), which contains a cell culture medium.

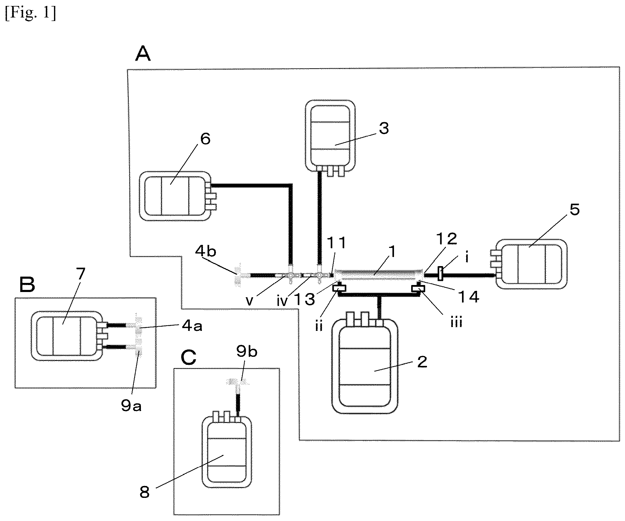

Examples of the device and kit of the present invention are shown in Fig. 1. The device of the present invention (part A) comprises at least the hollow fiber type separator (1), the drainage bag (2) connected to the discharge port of the hollow fiber type separator, the collection bag (3) connected to the inlet or outlet of the hollow fiber type separator, and the aseptic connection connector (4b) connected to the inlet of the hollow fiber type separator. In the embodiment shown in Fig. 1, it further comprises a bag containing liquid B (5) connected to the outlet port (12) of the hollow fiber type separator (1), and a bag containing liquid B (6) connected to the inlet port (11) of the hollow fiber type separator (1). In Fig. 1, there are further shown a bag containing suspension (7) provided with aseptic connection connectors (4a, 9a) as the part B, and a bag (8) provided with an aseptic connection connector (9b) and containing a liquid to be added to the bag containing suspension (7) as the part C. In the following descriptions, the present invention and embodiments thereof will be explained with reference to Fig. 1 with citing the numerals and symbols mentioned in Fig. 1 in parentheses, as required.

### [Hollow fiber type separator]

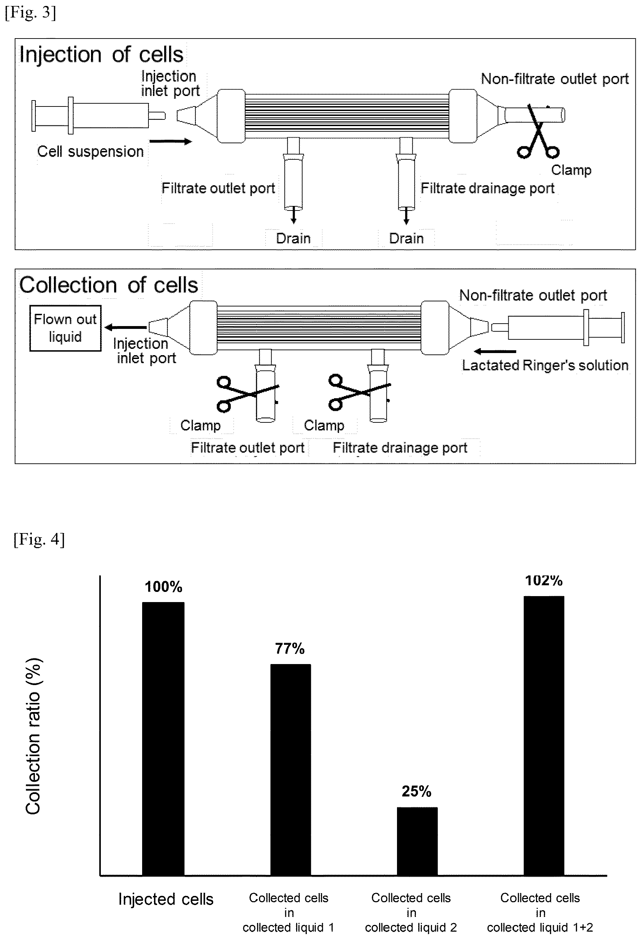

The device of the present invention comprises the hollow fiber type separator (1). The term hollow fiber type separator refers to a separator using a hollow fiber type separation membrane (also simply referred to as hollow fiber, a membrane in the form of straw having a large number of pores in the wall), and used for the purpose of separation, or concentration, molecular sieving, dialysis, liquid exchange, and so forth based on the separation ability. The hollow fiber type separator is typically the hollow fiber type separator shown at the centers of the upper and lower drawings of Fig. 3. The hollow fiber type separator consists of a straight body part and header parts provided at the both ends of the body part, the header parts have the inlet port (11) and outlet port (12), which communicate with the inside of the hollow fiber, and the body part has at least one discharge port (13, 14) which communicates with the outside of the hollow fiber. As is well known to those skilled in the art, in the inside of the body part of the hollow fiber type separator used in the present invention, from several tens to several thousands of bundled hollow fibers are filled, and it comprises a resin layer part that fixes a bunch of the fibers in the inside of a container, and forms open ends of the hollow fibers. Therefore, it has a structure that the inlet and outlet that communicate with the inside of the hollow fibers and the discharge port are separated by the hollow fiber type separation membranes.

According to the present invention, the suspension flows through the inside of the hollow fibers of the hollow fiber type separator, the suspending medium (liquid A) is filtered outside the hollow fibers, and thus the suspending medium is removed from the suspensoid. By using the hollow fiber type separator in this manner, a sufficiently high flow rate of the suspension is secured at the membrane surface, and the suspensoid hardly adheres to the membrane surface thanks to the cross flow (also called tangential flow) effect. Therefore, the suspending medium can be quickly removed without requiring high pressure load, thus damages on the suspensoid are suppressed, and the suspensoid can be concentrated even to a high concentration.

The hollow fibers used for the hollow fiber type separator are those having such an internal diameter that the suspensoid can pass thorough them, and a pore diameter that enables separation of the suspensoid and the suspending medium (liquid A). When the suspensoid consists of cells, the internal diameter can be 0.03 mm or larger, preferably 0.1 mm or larger, more preferably 0.3 mm or larger. Although the upper limit of the internal diameter is not particularly defined, from an aspect that a larger number of hollow fibers having a smaller diameter provide a larger membrane surface for the treatment and thus enable more efficient separation, those having an internal diameter of, for example, 2 mm or smaller, preferably 1 mm or smaller, can be used.

When the suspensoid consists of cells, it is sufficient that the pore diameter (also referred to as pore size) is smaller than the diameter of the cells. It may be, for example, 0.05 µm or larger and 10 µm or smaller, preferably 0.1 µm or larger and 5 µm or smaller, more preferably 0.15 µm or larger and 1 µm or smaller. If the pore diameter is too small, sufficient filtration velocity cannot be obtained. If it exceeds 10 µm, the cells to be treated may enter into the pores to reduce the collection ratio of the cells.

The pore diameter of the hollow fibers may be such a size that the objective cells are appropriately treated, i.e., the suspension (liquid A) can be filtered without passing the cells. Therefore, when an existing hollow fiber type separator is used, those using hollow fibers having comparatively large pore diameter called micro flow (MF) type, or those using, among hollow fibers having molecular fractionation ability called ultra flow (UF) type, such hollow fibers having comparatively large pore diameter (comparatively large molecular cutoff) can be selected. When those of the UF type are chosen, those having a molecular cutoff sufficient for passing an ingredient desired to be removed (for example, culture medium components, insulin, transferrin, serum albumin, and IL-2 for proliferation, induction of differentiation, and activation of cells) can be selected. When an existing hollow fiber type separator is used, it can be chosen on the basis of nominal pore size indicated by manufacturer. Alternatively, an average of diameters (length of major axis in the case of elliptic section) of 20 pores observed with a scanning electron microscope may be used as the basis of the selection.

It is preferred that all the suspensoid contained in the suspension to be treated can be contained in the inside of the hollow fibers of the hollow fiber type separator from the aspect of enabling efficient and sufficient treatment. When the device of the present invention is for treating cells for therapeutic use, it is thought that the number of cells treated by one time of the treatment operation is up to 2 x 10⁹ cells, and therefore a hollow fiber type separator having such a total internal volume of hollow fibers that the aforementioned amount of cells can be contained can be chosen. The total internal volume of the hollow fibers can be calculated in accordance with the following equation. Total internal volume of hollow fibers (mL or cm³) = [Internal diameter of hollow fibers (cm)/2]² x Π x length of hollow fibers (cm) x number of hollow fibers

Membrane area may be indicated as an index representing throughput of a hollow fiber type separator. The membrane area of a hollow fiber type separation membrane is usually calculated in accordance with the following equation. Membrane area (cm²) = Internal diameter of hollow fibers (cm) x Π x length of hollow fibers (cm) x number of fibers

According to the investigations of the inventors of the present invention, 1 x 10⁷ cells could be preferably treated with a hollow fiber type separator using hollow fibers having an internal diameter of 0.7 mm and a pore diameter of 0.25 µm, and having a membrane area of 150 cm². Since the total internal volume of this hollow fiber type separator is calculated to be about 2.6 mL, if the cell density can be concentrated to about 8 x 10⁸ cells/mL by separating the culture medium, the hollow fiber type separator can contain up to about 2.0 x 10⁹ of cells in the inside of the hollow fibers.

Therefore, if it is supposed to treat 1 x 10⁷ to 2 x 10⁹ cells, use of a hollow fiber type separator having a membrane area of 10 to 20,000 cm² enables favorable treatment. If a separator having a membrane area large to some extent is used, sufficient separation can be achieved, whereas if a separator having a membrane area smaller than a certain level is used, reduction of cell collection ratio and cost can be suppressed.

Although the material of the hollow fibers is not particularly limited so long as the objective separation can be performed, a material durable to sterilization is preferred. When it is used for treating cells for therapeutic use, it is preferably a material acceptable as a medical supply. Specifically, it is preferably a polymer material of polyolefine type or polysulfone type, and more specifically, it is preferably polyethylene (PE), polysulfone (PS), polyether sulphone (PES), modified polyether sulphone (mPES), or mixed cellulose ester (ME).

When sterilization is referred to for the present invention, the means therefor is not particularly limited, and examples include heat sterilization such as autoclaving and boiling, gas sterilization with a disinfectant gas such as ethylene oxide gas and hydrogen peroxide gas, radiation sterilization with ultraviolet ray, γ-ray, electron beam, and so forth. It is preferably a means acceptable as a sterilization means for a medical supply.

Although the material of the body part and header part of the hollow fiber type separator is not particularly limited so long as the objective separation can be performed, it is preferably a material transparent so that separation can be visually confirmed, and durable to sterilization. Specifically, it is preferably a block copolymer of polysulfone, polypropylene, polyvinyl chloride, polyethylene, polyimide, polycarbonate, polymethylpentene, polystyrene, and so forth. Although the material of the resin layer part that fixes the hollow fiber type separation membrane is not also particularly limited so long as the objective separation can be performed, it is preferably a material durable to sterilization. Specifically, it is preferably a polyurethane resin, epoxy resin, or silicone resin.

By using the hollow fiber type separator, the liquid A can be separated from a suspension containing the liquid A as a suspending medium and a suspensoid. When the device of the present invention is for treating cells for therapeutic use, the liquid A is usually a culture medium containing ingredients for proliferation, induction of differentiation, and activation of cells, and such ingredients are preferably sufficiently removed at the time of administration to a patient. According to the present invention, such ingredients can be sufficiently removed from cells for therapeutic use by using the hollow fiber type separator.

### [Bags]

The device of the present invention comprises the drainage bag (2) for storing the separated liquid A, which is connected to the drainage ports (13, 14) of the hollow fiber type separator (1), and the collection bag (3) for storing the suspensoid from which the liquid A has been separated, which is connected to the inlet port (11) or outlet port (12) of the hollow fiber type separator. When the device of the present invention is used for treating cells for therapeutic use to be intravenously administered by drip infusion, the collection bag (3) may be a bag suitable for drip infusion (drip infusion bag).

Although the collection bag (3) is connected to the inlet port (11) or the outlet port (12) of the hollow fiber type separator (1), it is preferably connected on the opposite side of one of the bag containing liquid B (5) explained later with respect to the hollow fiber type separator (1). Namely, it is preferred that when the collection bag (3) is connected to the inlet port (11) of the hollow fiber type separator (1), at least one bag containing liquid B (5) is connected to the outlet port (12) of the hollow fiber type separator (1), and when the collection bag (3) is connected to the outlet port (12) of the hollow fiber type separator (1), at least one bag containing liquid B (5) is connected to the inlet port (11) of the hollow fiber type separator (1). This is because, with such a configuration, the suspensoid from which the liquid A has been separated in the hollow fiber type separator (1) can be extruded by the liquid B, and stored in the bag for collection (3). The collection bag (3) is preferably connected via a connection part that can switch flow paths such as a three-way cock (iv).

The device of the present invention may comprise, besides the drainage bag (2) and the collection bag (3), at least one (for example, two) bag containing liquid B (5, 6), which is connected to the inlet port (11) or outlet port (12) of the hollow fiber type separator (1), and contains the liquid B for washing or collecting the suspensoid from which the suspending medium has been separated. Alternatively, it may comprise at least one (for example, two) connector for easily connecting the bag containing liquid B. Such a connector may be an aseptic connection connector for aseptic connection in a non-aseptic environment. The parts to which the hollow fiber type separator (1), the drainage bag (2), and the collection bag (3) are connected, and the bags containing liquid B (5, 6) can be separately sterilized by an appropriate sterilization means, and then aseptically connected.

The liquid B is a liquid used for washing the suspensoid, and/or replacing the liquid A to re-suspend the suspensoid. Although the liquid B can be chosen from various kinds of liquids depending on the purpose, when the device of the present invention is for treating cells for therapeutic use, as the liquid B for washing the cells, there can be used phosphate buffered saline (PBS), Tris buffered saline (TBS), HEPES buffered saline, physiological saline, Ringer's solution (lactated Ringer's solution, acetic acid Ringer's solution, bicarbonate Ringer's solution, etc.), 5% glucose aqueous solution, etc., which have a buffering effect. For re-suspending cells for drip infusion, a liquid acceptable for drip infusion is used. Examples of such a liquid include Ringer's solution (lactated Ringer's solution, acetic acid Ringer's solution, bicarbonate Ringer's solution, etc.), physiological saline, 5% glucose aqueous solution, and so forth.

Although the volume of the liquid B can be appropriately determined depending on the purpose of the use of the liquid B such as washing and re-suspension for drip infusion, it is usually about 0.5 to 4 times of that of the suspension to be treated with the hollow fiber type separator or the liquid A.

In a preferred embodiment, two or more of the bags containing liquid B (5, 6) are provided, at least one of them is connected to the inlet port (11) of the hollow fiber type separator (1), at least one of them is connected to the outlet port (12), one is used for washing the suspensoid, and the other is used for collection and re-suspension. By using separate liquid B for washing and liquid B for collection and re-suspension, washing can be performed with a sufficient volume of the liquid B, and the suspensoid can be collected in a state that the suspensoid is re-suspended in the liquid B of a correctly determined volume. With such a configuration, the liquid feeding for washing and the liquid feeding for collection are performed in opposite directions, and therefore increase of the collection ratio can be expected, and in addition, the connection parts of tubes can be reduced in the whole device, which provides merits in the manufacture of the device. The bag containing liquid B (6) connected to the inlet side of the hollow fiber type separator is preferably connected via a connection part that can switch flow paths such as a three-way cock (v).

The number, type (model), and capacity of the bags are not particularly limited, and can be appropriately determined depending on the volume of the liquid A to be separated, volume of the suspensoid to be stored, volume of the liquid B required, and so forth. For example, a pillow type bag, cylindrical tank type bag, container tank type bag, square-shaped tank type bag, and so forth can be used, and those having a capacity of, for example, 50 mL, 100 mL, 200 mL, 300 mL, 500 mL, 1 L, 5 L, 10 L, 20 L, 50 L, etc. can be appropriately used. For treating cells for therapeutic use, as the drainage bag (2), one having a capacity of 50 mL to 5 L can be chosen, and as the collection bag (3), one having a capacity appropriate for drip infusion, for example, about 50 mL to 1L, can be chosen. As the bags containing liquid B (5, 6), for example, those having a capacity of about 50 mL to 1 L can be chosen.

The material of the bags is not particularly limited so long as such a flexible resin material that the bags can be deformed when pressure is applied to them to extrude the content is chosen. However, it is preferably a material transparent or translucent so that transfer or storage of the liquid can be visually confirmed, and durable to sterilization. Specifically, there can be exemplified polyethylene, polypropylene, polybutadiene, ethylene-vinyl acetate copolymer (EVA), ethylene-vinyl alcohol copolymer resin (EVOH), polyvinyl chloride, and so forth. It is more preferred that the material of the bags is not a halogen type material, and is not a material using the plasticizer DEHP in consideration of the environment. When the device of the present invention is for treating cells for therapeutic use, the material of the bags is preferably a material approved as a material for medical supply.

### [Aseptic connection connector]

The device of the present invention comprises the aseptic connection connector (4b) for aseptically connecting the bag connected to the inlet port (11) of the hollow fiber type separator (1) and containing the suspension in a non-aseptic environment. The non-aseptic environment refers to a usual open environment not in a special facility such as clean room, for example, consultation room or ward of hospital or clinic, laboratory, home, and so forth.

The aseptic connection connector (4b) is a connector that can connect two tubes without exposing the inside of the tube to the external atmosphere and without requiring any special machinery such as aseptically connecting apparatus, and a marketed product can be used. The aseptic connection connector (4b) may be one of two members of an aseptic connection connector consisting of the two members, such as Medlock Connector and Kleenpak (registered trademark) Connector of PALL Corporation, Opta (registered trademark) STF-I Connector of Sartorius Stedim Biotech, Lynx (registered trademark) ST Connector of Millipore, and AseptiQuik (registered trademark) Connector of Colder Products Company.

Although the aseptic connection connector (4b) is not particularly limited so long as it enables the intended connection, it preferably consists of a material durable to sterilization suitable for the other parts such as the bags. The material of the aseptic connection connector (4b) can be, for example, polycarbonate, silicone rubber, polyethylene, or polyether sulphone.

### [Tubes]

In the device of the present invention, such parts as the hollow fiber type separator, bags, and aseptic connection connectors are liquid-tightly connected (connected in a state that liquid does not leak), and tubes can be used for the connection.

Although the material of the tubes is not particularly limited so long as it is a soft resin material that can liquid-tightly connect the hollow fiber type separator, bags, and connectors, it is preferably a material that can be sterilized with those materials, and is transparent or translucent so that transfer of the liquid can be visually confirmed. Specifically, there can be exemplified polyolefines such as polyethylene, polypropylene, ethylene-propylene copolymer, and ethylene-vinyl acetate copolymer (EVA), polystyrene, polyamide, polyimide, poly(4-methylpentene-1), ionomer, acrylic resin, polyesters such as polyethylene terephthalate (PET), and polybutylene terephthalate (PBT), various thermoplastic elastomers of styrene type, polyolefine type, polyvinyl chloride type, polyurethane type, polyester type, polyamide type, and so forth, silicone resin, polyurethane, and so forth as well as copolymers, blends, and polymer alloys containing any of those materials as a main component.

### [Other parts]

The device of the present invention may comprise, besides the aforementioned hollow fiber type separator, bags, and aseptic connection connectors, various kinds of members, for example, clamps, sampling port, cocks, clave connectors, and so forth, as required.

In a preferred embodiment, the device of the present invention comprises a first clamp (i) for closing the outlet port (12) of the hollow fiber type separator (1), and a second clamp (ii, iii) for closing the outlet of the hollow fiber type separator. When there are provided two or more of the outlets, two or more of the second clamps (ii, iii) may be provided to close them. The device of the present invention may comprise a three-way cock (iv, v) at a proper position for switching flow paths, or branching a flow path.

### [Part B]

The suspension containing the liquid A as the suspending medium, and the suspensoid may be stored in the bag (7) before it is treated with the device of the present invention. The kit provided by the present invention comprises, besides the part A consisting of the aforementioned device, a part consisting of the bag (7) containing suspension as the part B. The bag (7) containing suspension preferably comprises an aseptic connection connector (4a) for aseptic connection with the device of the present invention. The bag (7) containing suspension may also comprise a further aseptic connection connector (9a) for aseptic connection with a bag (8) containing another liquid.

### [Part C]

The kit of the present invention may comprise a bag (8) containing another liquid. The bag (8) containing another liquid preferably comprises an aseptic connection connector (9b) for aseptic connection with the bag (7) containing suspension. When the device of the present invention is for treating cells for therapeutic use, an example of the other liquid contained in the bag (8) is a liquid containing ingredients for culturing and activating the cells for therapeutic use, specifically, the aforementioned culture medium containing ingredients for proliferation, induction of differentiation, and activation of the cells (for example, serum, cytokines, growth factors, hormones, specifically, insulin, transferrin, serum albumin, IL-2 etc.).

In the kit, the parts A, B, and C are separately contained, and therefore each of them can be maintained at an optimal temperature until just before use. For example, the part A may be stored at ordinary temperature, the part B may be cryopreserved, and the part C may be refrigerated.

The kit may contain other components, besides the parts A, B, and C. For example, it may contain a medium recording information including connection methods, procedures for use, precautions, and so forth for the parts A, B, and C.

### <Method for preparing useful cells>

The present invention also provides a method for preparing useful cells aseptically separated from a liquid A from a suspension containing the liquid A as a suspending medium and the useful cells, which comprises the following steps:
- the step of feeding the suspension into a hollow fiber type separator (comprising an inlet and outlet for passing the suspension, which communicate with the inside of hollow fibers, and at least one discharge port for discharging the separated liquid A, which communicates with the outside of the hollow fibers) of which outlet is closed from the inlet of the hollow fiber type separator to separate the liquid A, and discharging the separated liquid A from the discharge port, so that the useful cells from which the liquid A has been separated remain in the inside of the hollow fibers; and
- the step of feeding a liquid B prepared beforehand to the hollow fiber type separator of which discharge port is closed from the outlet of the hollow fiber type separator to extrude the useful cells remained in the inside of the hollow fibers, and collecting the useful cells.

The present invention also provides a method for preparing useful cells aseptically separated from a liquid A from a suspension containing the liquid A as a suspending medium and the useful cells, which comprises the following steps:
(1) the step of aseptically connecting a bag containing suspension in which the suspension is retained to the aseptic connection connector of the aforementioned device of the present invention;
(2) the step of feeding the suspension into the hollow fiber type separator of which outlet is closed from the inlet of the hollow fiber type separator to separate the liquid A, so that the separated liquid A is stored in the drainage bag, and the useful cells from which the liquid A has been separated remain in the inside of the hollow fibers; and
(3) the step of feeding a liquid B prepared beforehand into the hollow fiber type separator of which discharge port is closed from the outlet of the hollow fiber type separator to extrude the useful cells in the inside of the hollow fibers, and storing them in the collection bag.

Examples of the useful cells include cells used for a treatment (prophylactic treatment, therapeutic treatment, etc.) of a disease or condition in a human or nonhuman animal (cells for therapeutic use). That is, the present invention also provides a method for preparing cells for therapeutic use from a cell suspension, which comprises the following steps (1) to (3):
(1) the step of aseptically connecting a bag containing cell suspension in which the cell suspension is retained to the aseptic connection connector of the aforementioned device of the present invention;
(2-1) the step of feeding the cell suspension into the hollow fiber type separator of which outlet is closed from the inlet of the hollow fiber type separator to separate culture medium, so that the separated culture medium is stored in the drainage bag, and the useful cells from which the culture medium has been separated remain in the inside of the hollow fibers;
(2-2) the step of feeding a liquid suitable for intravenous administration from the first bag containing liquid B into the hollow fiber type separator of which outlet is closed to wash the cells remained in the inside of the hollow fibers, from which cells the culture medium has been separated, and
(3) the step of feeding a liquid suitable for intravenous administration from the second bag containing liquid B into the hollow fiber type separator of which discharge port is closed to extrude the washed cells in the inside of the hollow fibers, and storing them in the collection bag to obtain the cells for therapeutic use.

The method may comprise the following step as a step preceding the step (1):
(0) the step of aseptically adding a culture medium to a cell storage bag containing frozen cells together with a preservation liquid to obtain a bag containing cell suspension, which retains the cell suspension.

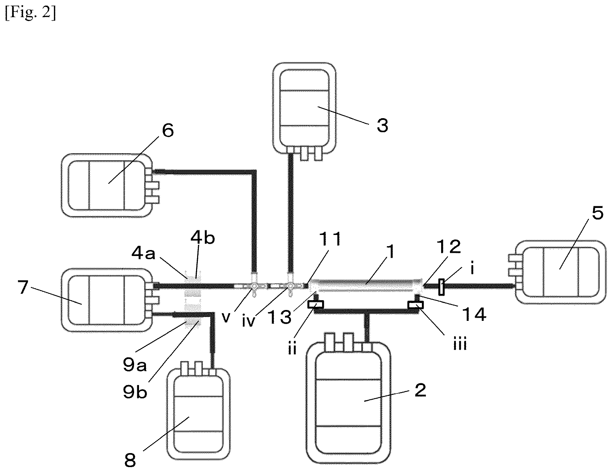

An example of the device of the present invention at the time of use for implementing the method for preparing useful cells of the present invention is shown in Fig. 2. In the following descriptions, the present invention and embodiments thereof will be explained with reference to Fig. 2 with citing the numerals and symbols mentioned in Fig. 2 in parentheses, as required.

The step (1) is a step of aseptically connecting a bag containing cell suspension in which the cell suspension is retained to the aseptic connection connector of the device of the present invention. Specifically, the bag containing cell suspension (7) is connected to the instrument provided with the aseptic connection connector (4b) using the aseptic connection connector (4a), and the treatment using the instrument is started.

The step (2) is a step of feeding the cell suspension into the hollow fiber type separator of which outlet is closed from the inlet of the hollow fiber type separator to separate the culture medium. Specifically, in a state that the three-way cocks (iv, v) are appropriately set, the bag containing cell suspension (7) is pressurized to feed the suspension into the hollow fiber type separator from the inlet port (11) of the hollow fiber type separator. At this time, by closing the flow path connected to the outlet port (12) of the hollow fiber type separator with a first clamp (i), at least a part of the liquid A as the suspending medium is passed thorough the hollow fiber membranes, and stored in the drainage bag (2) via the discharge ports (13, 14) of the hollow fiber type separator (1). On the other hand, the cells from which at least a part of the liquid A has been separated remain in the inside of the hollow fibers.

After the step (2), and before the step (3), a step for washing of the cells may be performed. Specifically, the flow path is switched by using the three-way cock (iv), and the bag containing liquid B (6) is pressurized to feed the liquid B into the hollow fiber type separator from the inlet port (11) of the hollow fiber type separator, and thereby wash the cells remaining in the inside of the hollow fibers with the liquid B. The liquid B passes through the hollow fiber membranes, and stored in the drainage bag (2) via the drainage ports (13, 14) of the hollow fiber type separator (1).

The step (3) is a step of feeding the liquid B into the hollow fiber type separator of which discharge port is closed from the outlet of the hollow fiber type separator to extrude the useful cells in the inside of the hollow fibers, and storing them in the collection bag. Specifically, the first clamp (i) is released, the flow path is switched by using the three-way cock (v), and the bag containing liquid B (5) is pressurized to feed the liquid B into the hollow fiber type separator from the outlet port (12) of the hollow fiber type separator. At this time, by closing the flow paths connected to the discharge ports (13, 14) of the hollow fiber type separator with the second clamps (ii, iii), the cells in the inside of the hollow fibers are extruded with the liquid B, pass through the inlet port (11) of the hollow fiber type separator (1), and stored in the collection bag (3). In the cell suspension stored in the collection bag (3), the liquid A as the suspending medium has been replaced with the liquid B.

When cells for therapeutic use are prepared, the bag (7) containing suspension contains cells for therapeutic use, and it may be frozen for preservation or transportation, thawed, cultured again, and subjected to a treatment such as activation before administration in a medical facility in which a patient is present. In such a case, the step (0) of aseptically adding the culture medium to the cell preservation bag containing the cells frozen together with a preservation liquid can be performed before the step (1). Specifically, the bag (7) containing the suspension is aseptically connected to a bag (8) containing a solution for culture and activation using the aseptic connection connectors (9a, 9b), and the solution for culture and activation is injected into the bag (7) containing the suspension.

In the present invention, the liquids can be fed by pressurizing the bags containing the liquids. For the pressurization, an instrument that can adjust volume of the liquid by applying a constant pressure to the bag from the outside can be used. Such an instrument is called pressure bag or disposable pressure bag, and is marketed. Commercial pressure bags are usually provided with a pressure meter, and they enables liquid feeding with confirming degree of pressurization.

### <Use and others>

The present invention relates to aseptic separation of a liquid A from a suspension containing the liquid A as a suspending medium, and a suspensoid, and examples of the suspensoid include useful cells preferably used in research, inspection, therapeutic treatment, production of useful material, and so forth. Although the useful cells are not particularly limited, specific examples thereof include induced pluripotent stem cells (iPS cells), embryonic stem cells, hematopoietic stem cells, bone marrow stroma cells, mesenchymal stem cells, adipose-derived mesenchymal cells, adipose-derived stromal stem cells, pluripotent adult stem cells, lymphocyte type cells such as T cells, B cells, killer T cells (cytotoxic T cells), NK (natural killer) cells, NKT (natural killer T) cells, and regulatory T cells, macrophages, monocytes, dendritic cells, granulocytes, erythrocytes, thrombocytes, somatic cells such as nerve cells, myocytes, fibroblasts, hepatocytes, and cardiac muscle cells, and cells subjected to such a treatment as genetic transfection, and induction of differentiation.

The present invention is suitable for treating, among useful cells, especially cells for therapeutic use used for a treatment of a disease or condition of a human or nonhuman animal. Example of the cells for therapeutic use include dendritic cells, NK cells, NKT cells, activated T cells (including Th1 cells, Th2 cells, T_{FH} cells, Thl7 cells, Treg cells, etc., which constitute a functional subgroup of the effecter T cells produced as a result of activation of naive T cells at the time of encountering an antigen), and CAR-T cell (chimeric antigen receptor T cell).

Examples of the disease or condition to which cells for therapeutic use prepared according to the present invention can be applied include cancers, infectious diseases, autoimmune diseases, allergies, and so forth.

### Example

The K562 cells (human leukemic cell strain) were cultured in the RPMI1640 medium (Wako Pure Chemical Industries, catalog number 189-02025) containing 10% fetal calf serum (Nichirei Bioscience, catalog number 171012-500ML), 100 units of penicillin, and 100 µg/mL of streptomycin (Nacalai Tesque, catalog number 26253-84) (henceforth referred to as medium). The cell number of the K562 cells was counted by using the Neubauer counting plate (Erma Inc., catalog number 03-202-1), and 10⁷ of the K562 cells were suspended in 30 mL of the medium to prepare a cell suspension.

This cell suspension (30 mL) was injected into a hollow fiber module, Microza Pencil Type Module (Asahi Kasei Chemicals, ASA PMP-003, using polyethylene hollow fibers, hollow fiber internal diameter 0.7 mm, effective membrane area 150 cm², nominal diameter 0.25 µm) from the injection inlet port on one side by using a 50 mL syringe (TERUMO, catalog number SS-50ESZ). At the time of this operation, the non-filtrate outlet port on the opposite side of the injection inlet port was closed by attaching a tube (thermoplastic elastomer tube, C-Flex 374-500-3, Saint-Gobain) thereto and pinching it with a clamp, tubes (C-Flex 374-125-2, Saint-Gobain) were attached to the filtrate outlet port and the filtrate drainage port, and the other ends of these tubes were put into a drainage container to collect the drain (refer to Fig. 3, upper drawing).

Then, the filtrate outlet port and the filtrate drainage port were closed by using clamps, the tube attached to the non-filtrate outlet port was removed, and then a 50 mL syringe containing 50 mL of lactated Ringer's solution (Fuso Pharmaceutical Industries, Ltd., JAN Code 4 987197 900152) was directly connected. By pushing the plunger to inject the lactated Ringer's solution, and the liquid flown out from the injection inlet port was collected (referred to as collected liquid 1) (refer to Fig. 3, lower drawing). The cells in the collected liquid 1 was diluted 2 times by using a 0.4% Trypan Blue solution (Life Technologies, catalog number 15250-061), and counted by using the Neubauer counting plate. As a result, it was found that 50 mL of a cell suspension containing 1.55 x 10⁵ cells/mL was collected (7.75 x 10⁶ cells, collection ratio 77.5%).

The lactated Ringer's solution (50 mL) was further injected from the non-filtrate outlet port side by using a 50 mL syringe, and the liquid flown out from the injection inlet port was collected (referred to as collected liquid 2). The cells in the collected liquid 2 were counted by using the Neubauer counting plate. As a result, it was found that 50 mL of a cell suspension containing 5.0 x 10⁴ cells/mL was collected (2.50 x 10⁶ cells, collection ratio 25.0%).

The total of the collection ratios for the collected liquids 1 and 2 was about 100%, and thus all the injected cells were collected. The results are shown in the following table and Fig. 4.

**Table 1]**

| | Cell number | Collection ratio |
|---|---|---|
| Injected cells | 1.00E+07 | 100% |
| Cells collected from collected liquid 1 | 7.75E+06 | 77% |
| Cells collected from collected liquid 2 | 2.50E+06 | 25% |
| Cells collected from collected liquids 1 and 2 | 1.03E+07 | 102% |

### Description of Notations

- 1:: Hollow fiber type separator
- 2:: Drainage bag
- 3:: Collection bag
- 4:: Aseptic connection connector
- 5, 6:: Bag containing liquid B
- 7:: Bag containing suspension
- 8:: Other bag containing liquid (culture medium)
- 11:: Inlet port
- 12:: Outlet port
- 13, 14:: Drainage port
- i:: First clamp,
- ii, iii:: Second clamp
- iv, v:: Three-way cock
- A:: Part consisting of instrument
- B:: Part consisting of bag containing suspension
- C:: Part consisting of bag containing other liquid (culture medium)

## Claims

1. A device for aseptically separating a liquid A from a suspension containing the liquid A as a suspending medium and a suspensoid, the device comprising at least the followings:
- a hollow fiber type separator for separating the liquid A from the suspension, the hollow fiber type separator comprising
an inlet and outlet for passing the suspension, which communicate with the inside of hollow fibers, and
at least one discharge port for discharging the separated liquid A, which communicates with the outside of the hollow fibers;
- an aseptic connection connector for aseptically connecting a bag containing the suspension in a non-aseptic environment, which is connected to the inlet of the hollow fiber type separator;
- a drainage bag for storing the separated liquid A, which is connected to the discharge port of the hollow fiber type separator; and
- a collection bag for storing the suspensoid from which the liquid A has been separated, which is connected to the inlet or outlet of the hollow fiber type separator.

2. The device according to claim 1, which further comprises the following:
- at least one bag containing liquid B, which contains a liquid B for washing or collecting the suspensoid from which the suspending medium has been separated, and is connected to the inlet or outlet of the hollow fiber type separator.

3. The device according to claim 2, which comprises two bags containing liquid B.

4. The device according to claim 2 or 3, wherein a first bag containing liquid B is connected to the inlet of the hollow fiber type separator, and a second bag containing liquid B is connected to the outlet of the hollow fiber type separator.

5. The device according to any one of claims 1 to 4, which further comprises:
- a first clamp for closing the outlet of the hollow fiber type separator, and
- a second clamp for closing the discharge port of the hollow fiber type separator.

6. The device according to any one of claims 1 to 5, wherein:
the device is for aseptically separating a culture medium from a cell suspension containing cells as the suspensoid and the culture medium as the liquid A, and
the hollow fibers of the hollow fiber type separator have such an internal diameter that the cell suspension can pass through them, and such a pore diameter that the cells and the culture medium can be separated.

7. The device according to claim 6, wherein:
the cells are cells for therapeutic use to be intravenously administered, the device is for aseptically substituting a liquid suitable for intravenous administration for the culture medium of the cell suspension;
the device comprises a first bag containing liquid B connected to the inlet of the hollow fiber type separator, and a second bag containing liquid B connected to the outlet of the hollow fiber type separator, and the liquids B is the liquid suitable for intravenous administration.

8. A kit for preparing cells for therapeutic use, the kit comprising the followings:
- the device according to claim 7; and
- a bag containing stored cells, which contains useful cells together with a preservation liquid, and provided with at least one aseptic connection connector.

9. The kit according to claim 8, which further comprises the following:
- a bag containing culture medium, which contains a cell culture medium.

10. The kit according to claim 8 or 9, wherein the cells are any cells selected from the group consisting of dendritic cells, NK cells, NKT cells, activated T cells, and CAR-T cells.

11. A method for preparing useful cells aseptically separated from a liquid A from a suspension containing the liquid A as a suspending medium and the useful cells, the method comprising the following steps:
(1) the step of aseptically connecting a bag containing suspension in which the suspension is retained to the aseptic connection connector of the device according to any one of claims 1 to 7;
(2) the step of feeding the suspension into the hollow fiber type separator of which outlet is closed from the inlet of the hollow fiber type separator to separate the liquid A, so that the separated liquid A is stored in the drainage bag, and the useful cells from which the liquid A has been separated remain in the inside of the hollow fibers; and
(3) the step of feeding a liquid B prepared beforehand into the hollow fiber type separator of which discharge port is closed from the outlet of the hollow fiber type separator to extrude the useful cells in the inside of the hollow fibers, and storing them in the collection bag.

12. A method for preparing cells for therapeutic use from a cell suspension, the method comprising the following steps:
(1) the step of aseptically connecting a bag containing cell suspension in which a cell suspension is retained to the aseptic connection connector of the device according to claim 7;
(2) the step of feeding the cell suspension into the hollow fiber type separator of which outlet is closed from the inlet of the hollow fiber type separator to separate the culture medium, so that the separated culture medium is stored in the drainage bag, and the cells from which the culture medium has been separated remain in the inside of the hollow fibers;
(3) the step of feeding a liquid suitable for intravenous administration from the first bag containing liquid B into the hollow fiber type separator of which outlet is closed to wash the cells remained in the inside of the hollow fibers, from which cells the culture medium has been separated, and
(4) the step of feeding a liquid suitable for intravenous administration from the second bag containing liquid B to the hollow fiber type separator of which discharge port is closed to extrude the washed cells in the inside of the hollow fibers, and storing them in the collection bag to obtain the cells for therapeutic use.

13. The method according to claim 12, which further comprises the following step as a step preceding the step (1):
(0) the step of aseptically adding a culture medium to a cell storage bag containing frozen cells together with a preservation liquid to obtain a bag containing cell suspension, which retains the cell suspension.

14. The method according to claim 13, wherein the cells are any cells selected from the group consisting of dendritic cells, NK cells, NKT cells, activated T cells, and CAR-T cells.
